# FASCICULE DE BREVET EUROPEEN

(11) **EP 3 860 532 B1**
(45) Date de publication et mention de la délivrance du brevet: **22.02.2023**
(21) Numéro de dépôt: 19780213.5
(22) Date de dépôt: 27.09.2019
(51) Int. Cl.: A61F 5/08

(54) **CONFORMATEUR NARINAIRE**
NASENLOCHFORMER
NOSTRIL SHAPER

(30) Priorité: 01.10.2018 FR 1859085
(43) Date de publication de la demande: 11.08.2021
(73) Titulaire: Bone 3D, 75013 Paris (FR); Assistance Publique-Hôpitaux de Paris, 75004 Paris (FR)
(72) Inventeur: ADAM, Jérémy, 75013 PARIS (FR); PERRIN, Benoît, 92140 CLAMART (FR); KHONSARI, Roman Hossein, 75005 PARIS (FR)
(74) Mandataire: LLR
(86) Numéro de dépôt international: PCT/EP2019/076319
(87) Numéro de publication internationale: WO 2020/070028

(56) Documents cités:
- EP-A1- 1 917 993
- WO-A1-2017/020068
- US-A- 5 665 104
- US-A1- 2004 089 303
- US-A1- 2006 085 027

## Description

L'invention concerne un conformateur narinaire, destiné à conformer les narines, en particulier après un traitement de rhinoplastie.

On connaît déjà des conformateurs narinaires, comme par exemple celui décrit dans le brevet RU 2 477 088, utilisés après une rhinoplastie, et en particulier en cas de chirurgie reconstructrice d'une fente labio-palatine chez l'enfant. Ces conformateurs fonctionnent de manière relativement satisfaisante. On sait par ailleurs que toute cicatrice peut avoir un aspect peu esthétique du fait de bourrelets ou de creux qui se créent lors de la cicatrisation. Ceci est particulièrement gênant pour une cicatrice au visage, disposée dans la zone s'étendant entre la lèvre et le nez, c'est-à-dire dans la zone du philtrum. Or, le conformateur narinaire décrit dans le brevet RU 2 477 088 ne permet pas d'obtenir chez tous les patients une cicatrice ayant un aspect esthétique satisfaisant.

On connaît également du document US 5 665 104 A, un article inséré dans chaque narine du nez d'un humain dans le but d'augmenter l'espace de respiration de celui-ci. L'article comprend deux pièces substantiellement cylindriques fixées l'une à l'autre par une bande de liaison sur leurs bases respectives.

L'invention a notamment pour but de fournir un conformateur narinaire permettant d'améliorer la cicatrisation après une rhinoplastie, tout en garantissant la restauration de la respiration nasale et en luttant contre la création d'oedèmes.

A cet effet l'invention a pour objet un conformateur narinaire configuré pour être introduit dans le nez d'un sujet et comprenant
- deux tubes aptes à être introduits chacun dans une narine du nez, reliés entre eux à leurs extrémités inférieures par un pont de liaison et
- une plaquette attachée au pont de liaison et s'étendant à l'opposé du pont de liaison par rapport aux tubes, la plaquette étant configurée pour exercer une pression sur le philtrum du sujet lorsque les tubes sont introduits chacun dans une narine du nez.

Ainsi, on propose un conformateur narinaire qui comprend une plaquette conçue de manière à appuyer sur le philtrum après que le conformateur a été mis en place dans les narines. Grâce à cette pression exercée sur le philtrum, la plaquette assure non seulement une protection d'une cicatrice disposée sur le philtrum, mais peut en outre exercer une pression suffisante sur la cicatrice pour éviter un bourrelet ou un creux cicatriciel. L'action de cette plaquette obéit donc au principe de la pressothérapie, dont les bénéfices sur les cicatrices chirurgicales sont reconnus.

On notera que le conformateur est particulièrement intéressant par cet aspect de pression sur la cicatrice : on ne se contente pas de protéger la cicatrice, on assure une pression qui permet de contrôler le processus de cicatrisation. On comprend donc que la plaquette proposée est configurée pour être en contact surfacique avec le philtrum.

On entend ici par « extrémité inférieure » des tubes l'extrémité qui est située à l'extrémité inférieure des narines lorsque le conformateur est en place.

Le conformateur narinaire peut en outre comporter les caractéristiques suivantes, prises seules ou en combinaison :
- La plaquette est attachée au pont de liaison en étant déformable entre :
   - une position de repos, dans laquelle la plaquette s'étend vers l'arrière du conformateur narinaire, en regard des tubes avant la mise en place du conformateur, et
   - une position d'utilisation, lorsque le conformateur narinaire est introduit dans le nez, dans laquelle la plaquette s'étend vers l'avant du conformateur narinaire.

Cette configuration de la plaquette mobile entre deux postions est particulièrement avantageuse pour exercer une pression sur le philtrum.
- Le conformateur narinaire comprend des moyens de rigidification, exerçant un rappel de la plaquette vers sa position de repos, par exemple un insert. Les moyens de rigidification peuvent être venus de matière avec la plaquette ou rapportés sur la plaquette. Ils peuvent par ailleurs être de la même matière que la plaquette ou de matière différente. De tels moyens de rigidification améliorent encore la pression exercée par la plaquette sur le philtrum, en augmentant le rappel de la plaquette vers sa position de repos.
   - Chaque tube comprend dans sa partie avant supérieure au moins une protubérance de positionnement du tube dans la narine. La ou les protubérances sont ainsi positionnées dans l'extrémité avant supérieure de la narine, ce qui permet un meilleur maintien du conformateur.
   - Chaque tube comprend dans sa partie arrière au moins une protubérance de calage du tube dans les fosses nasales du sujet, et de préférence deux protubérances de calage dans les fosses nasales du sujet. Ce mode de réalisation est particulièrement avantageux. En effet, on comprend que lorsque le conformateur est en place dans les narines, la protubérance est positionnée sous le cornet, ce qui permet un positionnement et un maintien optimal du conformateur. Il se trouve que l'on a constaté sur les conformateurs existants, en particulier celui proposé dans la publication RU2477088, qui ont une partie arrière dépourvue d'une telle protubérance, que le conformateur ne tient pas bien en place dans les narines car il a tendance à glisser vers le bas, ce qui oblige souvent à le suturer dans le nez du sujet, tout particulièrement pour un enfant. On comprend que la protubérance correspond à une partie convexe, faisant saillie de la surface arrière du tube. Lorsque le tube comprend deux protubérances, il comprend en fait deux parties convexes séparées par une partie concave.
   - L'extrémité inférieure de chaque tube présente un décrochement de façon à laisser un espace entre l'extrémité inférieure du tube et le bas de la narine, de préférence, ce décrochement présente la forme générale d'une marche d'escalier, pourvue de congés permettant d'adoucir les angles, reliant l'arrière et la partie inférieure du tube. On a ainsi une forme ne comprenant pas d'angle saillant, ce qui évite un frottement sur les points de suture placés à l'entrée des narines, qui peut être douloureux et/ou endommager les points.
- Le conformateur narinaire comprend en outre des languettes externes de maintien du conformateur sur le nez, s'étendant vers le haut depuis l'extrémité inférieure des tubes, de préférence deux languettes latérales destinées à se plaquer sur les ailes du nez et/ou une languette avant destinée à se plaquer sur la pointe du nez. La présence de ces languettes améliore la tenue du conformateur narinaire dans le nez, et peut avantageusement permettre d'appliquer une pression sur les différentes couches tissulaires des ailes nasales, pouvant être décollées durant une opération, améliorant ainsi la cicatrisation et permettant de maintenir la forme désirée par un chirurgien pendant l'opération. Ces languettes peuvent également servir à porter des points de sutures posés à travers les alaires, améliorant encore plus le contact des différentes couches tissulaires des ailes nasales.
- Le conformateur narinaire comprend du silicone, il est de préférence constitué de silicone.
   - La plaquette et/ou les éléments de rigidification sont fabriqués en un matériau plus rigide que le matériau des tubes.
   - Le conformateur narinaire comprend des couleurs de matériau différentes. Ainsi, il est possible de réaliser le conformateur dans des couleurs correspondant au mieux à la couleur de peau du patient, on peut également réaliser des décorations sur le conformateur.
- Le conformateur narinaire dispose d'une longueur de pont de liaison, ou columellaire, variable. La longueur variable de son pont de liaison permet que le conformateur narinaire peut s'adapter aux différences anatomiques, par exemple ethniques, des humains.

L'invention a également pour objet un conformateur narinaire comprenant deux tubes aptes à être introduits chacun dans une narine du nez, reliés entre eux à leurs extrémités inférieures par un pont de liaison, chaque tube comprenant dans sa partie arrière au moins une protubérance de calage du tube dans les fosses nasales du sujet, de préférence deux protubérances de calage dans les fosses nasales du sujet.

Comme cela a été présenté précédemment, on comprend qu'un tel conformateur narinaire répond au problème d'assurer une meilleure retenue du conformateur dans la narine, du fait que grâce à la ou les protubérances, on peut éviter d'avoir à suturer le conformateur dans la narine pour le faire tenir. Un tel conformateur narinaire peut comprendre l'une ou plusieurs des caractéristiques du conformateur narinaire présenté précédemment, prises seules ou en combinaison. On comprend en particulier que le conformateur narinaire peut comporter la plaquette attachée au pont de liaison, ou ne pas en comporter.

Un autre objet de l'invention est un procédé de fabrication d'un conformateur narinaire tel que décrit précédemment, comprenant une étape de moulage par injection, et le moule étant de préférence réalisé par fabrication additive.

La fabrication additive permet avantageusement de réaliser un moule sur mesure, donc un conformateur narinaire sur mesure, ce qui est particulièrement intéressant pour garantir une bonne tenue et une bonne pression sur le philtrum.

### Brève description des figures

L'invention sera mieux comprise à la lecture de la description qui va suivre donnée uniquement à titre d'exemple et faite en se référant aux dessins annexés dans lesquels :
- la [Fig. 1] est une vue en perspective isométrique d'un conformateur narinaire selon un premier mode de réalisation de l'invention,
- la [Fig. 2] est une vue latérale du conformateur narinaire de la [Fig. 1],
- la [Fig. 3] est une vue de dessous du conformateur narinaire de la [Fig. 1],
- la [Fig. 4] est une vue de face d'un conformateur narinaire selon un second mode de réalisation de l'invention,
- la [Fig. 5] est une vue en perspective du conformateur narinaire de la [Fig. 2],
- la [Fig. 6] est une vue latérale du conformateur narinaire de la [Fig. 2],
- la [Fig. 7] est une vue en perspective d'un moule pour la fabrication d'un conformateur narinaire similaire à celui de la [Fig. 1].

### Description détaillée

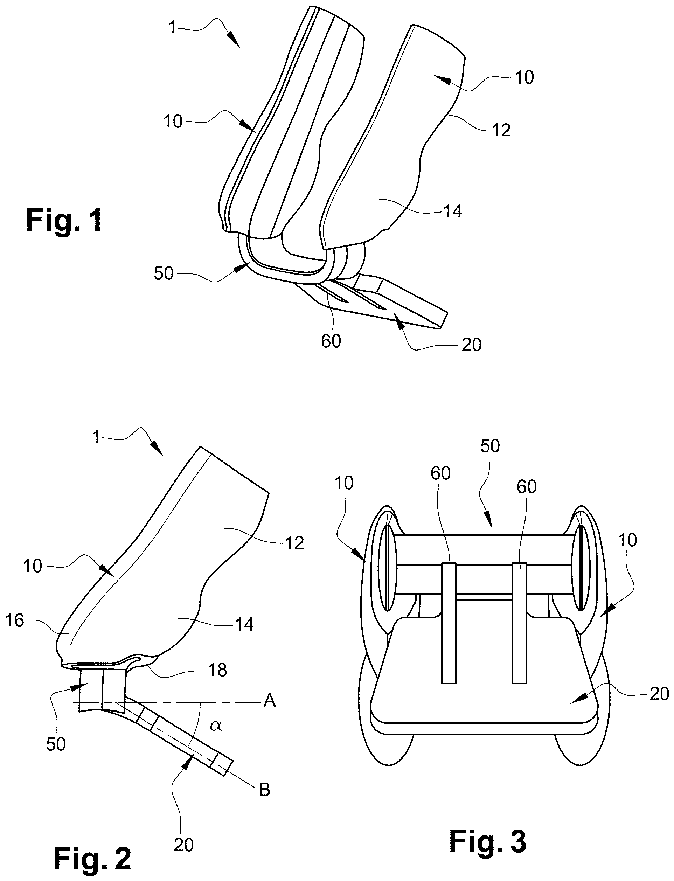

On a représenté sur les figures 1 à 3 un conformateur narinaire selon un premier mode de réalisation de l'invention, désigné par la référence générale 1.

De tels conformateurs narinaires sont généralement utilisés après une rhinoplastie. Le cas le plus fréquent d'utilisation d'un conformateur narinaire 1 est la suite d'une chirurgie reconstructrice d'une fente labio-palatine chez un enfant. Dans les suites de ce type de chirurgie, l'utilisation du conformateur narinaire a pour objectif de former les narines du sujet, selon une forme prédéfinie par le chirurgien, tout en lui permettant de respirer, et également, surtout chez un nourrisson, de se nourrir en étant capable de téter aisément. D'autre part, comme cela est exposé en détail ci-dessous, et selon le principe de la pressothérapie, le présent conformateur narinaire possède l'avantage de permettre d'obtenir une cicatrice ayant un meilleur aspect que celles obtenues avec les conformateurs de l'état de la technique.

Le conformateur narinaire 1 représenté sur les figures 1 à 3 comporte deux tubes 10 destinés à être introduits dans les narines d'un sujet. Les tubes 10 sont reliés entre eux par un pont de liaison 50. Lorsque les tubes 10 sont en place dans les narines du sujet, le pont de liaison 50 vient en général sensiblement en contact avec la base du nez du sujet.

Dans le mode de réalisation illustré par les figures 1 à 3, le conformateur narinaire 1 comporte en outre une plaquette 20 qui est attachée au pont de liaison 50. La plaquette 20 est configurée de façon à ce que, lorsque le conformateur narinaire 1 est en place dans le nez d'un sujet, c'est-à-dire lorsque les tubes 10 sont introduits chacun dans une narine du sujet, la plaquette 20 est positionnée à plat sur le philtrum du sujet, et exerce une pression sur le philtrum. Cette pression va contribuer à obtenir une cicatrice sur le philtrum qui possède un aspect esthétique satisfaisant.

Avantageusement, la plaquette 20 est mobile entre une position de repos et une position d'utilisation. Dans sa position de repos, la plaquette 20 s'étend vers l'arrière du conformateur narinaire 1, en regard des tubes 10. Cette position de repos de la plaquette 20 est généralement la position de la plaquette 20 obtenue en sortie de fabrication du conformateur narinaire. L'angle α formé par le plan de la plaquette et le plan passant par la partie inférieure du pont de liaison 50, et visible sur la figure 2 est compris entre 0° et 90°.

Dans la position d'utilisation de la plaquette 20, correspondant à la position dans laquelle le conformateur narinaire 1 est en place dans le nez du sujet, la plaquette 20 s'étend vers l'avant du conformateur narinaire 1, à l'opposé des tubes 10 par rapport au pont de liaison 50. La plaquette 20 est configurée de manière telle que lorsque le conformateur narinaire 1 est en place sur le nez du sujet la plaquette exerce une pression considérée comme suffisante pour améliorer la cicatrisation.

Avantageusement, ainsi que cela est illustré à la figure 3, le conformateur narinaire 1 comprend des moyens de rigidification 60 qui augmentent le rappel de la plaquette 20 de sa position d'utilisation vers sa position de repos. Ces moyens de rigidification 60 peuvent être des inserts placés dans la partie inférieure de la plaquette 20 et du pont de liaison 50, ou encore être venus de matière avec le reste de la plaquette 20. Ces moyens de rigidification 60 peuvent constituer, au moins en partie, les éléments d'attache de la plaquette 20 sur le pont de liaison 50. La présence de ces moyens de rigidification 60 améliore l'action de la plaquette 20 en augmentant la pression qu'elle exerce sur le philtrum du sujet.

Dans un mode de réalisation, les tubes 10 du conformateur narinaire 1 comprennent une protubérance 16, visible sur la figure 2, située dans la partie avant et supérieure du tube 10 et destinée à être placée dans la partie avant et supérieure d'une narine, ce qui présente l'avantage d'améliorer le maintien du conformateur narinaire 1 dans le nez du sujet.

Dans un autre mode de réalisation, chacun des tubes 10 est pourvu d'au moins une protubérance de calage 12, 14 telles que représentées en particulier sur les figures 1 et 2, et placée dans la partie arrière du tube. Ces protubérances de calage sont positionnées dans les fosses nasales du sujet lorsque le conformateur narinaire est en place, et plus précisément sous le cornet. La présence de ces protubérances de calage 12, 14 permet un positionnement et un maintien optimal du conformateur narinaire. Cela peut permettre en particulier d'éviter de suturer le conformateur narinaire dans le nez du sujet, ce qui était un inconvénient fréquent et important des conformateurs existants.

Dans encore un autre mode de réalisation, chaque tube 10 présente à sa extrémité inférieure avant, un décrochement 18, visible sur la figure 2, de manière telle qu'un espace est laissé entre l'extrémité inférieure du tube 10 et le bas de la narine. Avantageusement ce décrochement présente la forme générale d'une marche d'escalier, pourvue de congés permettant d'adoucir les angles. Autrement dit, le décrochement présente une section ayant sensiblement la forme d'un « S » dont les extrémités sont tangentes respectivement au tube 10 et au pont de liaison 50.

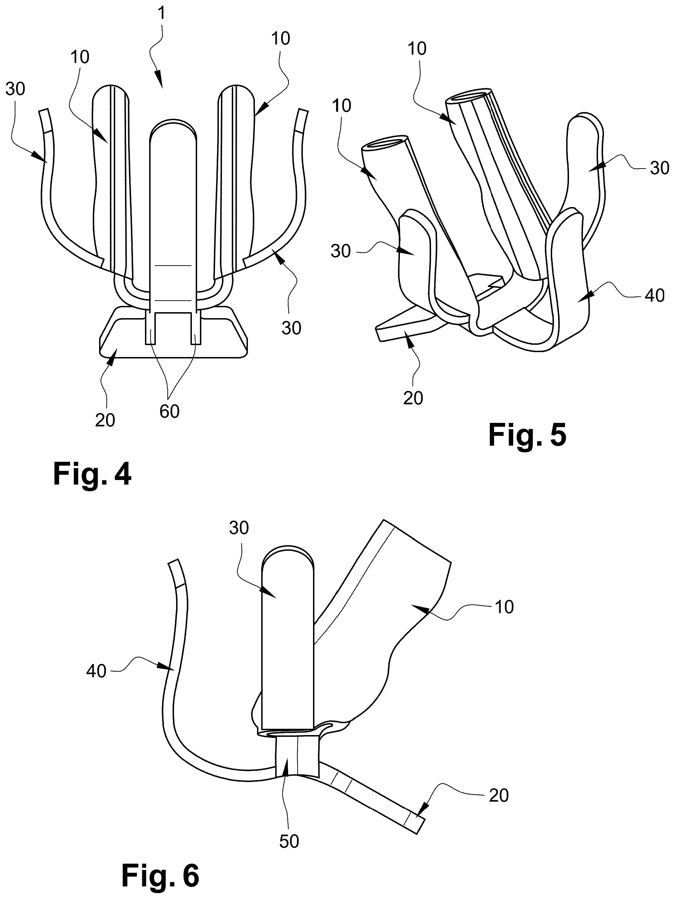

Dans un autre mode de réalisation illustré par les figures 4 à 6, le conformateur narinaire 1 comprend en outre des languettes externes de maintien 30, 40, destinées à maintenir le conformateur narinaire 1 en place dans le nez du sujet. Ces languettes 30, 40 s'étendent vers le haut depuis l'extrémité inférieure des tubes 10. Ces languettes peuvent être deux languettes externes latérales 30 destinées à se plaquer sur les ailes du nez, chacune d'un côté. Il peut également s'agir d'une languette avant 40, destinée à se plaquer sur la pointe du nez. Ces languettes latérales 30 et avant 40 peuvent être utilisées seules ou en combinaison.

En plus d'améliorer le maintien du conformateur narinaire 1 dans le nez du sujet, ces languettes permettent d'appliquer une pression sur les différentes couches tissulaires des ailes nasales, qui sont généralement décollées pendant la chirurgie. Ceci permet d'améliorer la cicatrisation, et en outre de maintenir la forme donnée aux narines par le chirurgien pendant l'intervention. Enfin, ces languettes peuvent servir de support à des points de sutures posés à travers les alaires, améliorant encore davantage le contact entre les différentes couches tissulaires des ailes nasales.

Dans les suites d'interventions de reconstruction labio-palatines, le chirurgien utilise avantageusement, dans les premiers jours qui suivent l'intervention, un conformateur narinaire 1 ne comprenant pas les languettes 30, 40, puis pendant les quelques semaines qui suivent, un conformateur narinaire 1 avec les languettes externes latérales 30 et / ou la languette externe avant 40. Ceci permet d'obtenir une meilleure cicatrisation des narines, et de bien conformer les narines selon la forme souhaitée.

Dans un autre mode de réalisation, la longueur du pont de liaison 50 est variable, et peut être adaptée à l'anatomie de chaque sujet.

De préférence, les tubes 10 sont fabriqués en un matériau flexible, de manière à pouvoir être introduits dans la narine plus facilement et en causant moins d'inconfort au sujet. Le silicone est un matériau particulièrement approprié pour les tubes 10. En effet, il possède une bonne flexibilité, et il est biologiquement inerte et bien toléré par le corps. De même, le pont de liaison 50 est de préférence flexible, et le silicone convient particulièrement bien. Avantageusement, le pont de liaison 50 est venu de matière avec les tubes 10.

De préférence la plaquette 20 comprend également du silicone. D'autre part, la plaquette 20 est configurée pour permettre d'appliquer une pression suffisante et sensiblement uniforme sur le philtrum, et plus particulièrement sur la zone du philtrum où se situe la cicatrice, et pour cela elle possède une rigidité appropriée, c'est-à-dire relativement élevée.

Ainsi, avantageusement, la plaquette 20 est plus rigide que les tubes 10 et le pont de liaison 50. En effet, dans le but d'augmenter la facilité d'utilisation et le confort, il est utile que les tubes 10 soient très flexibles. En revanche, pour fonctionner de manière satisfaisante, il est nécessaire que la plaquette 20 soit rigide.

Les moyens de rigidification 60 comprennent également avantageusement du silicone, voire sont constitués de silicone. Du fait qu'ils doivent contribuer à augmenter le rappel exercé sur la plaquette 20, ils possèdent généralement une rigidité relativement élevée, par exemple la même rigidité que celle de la plaquette 20. Dans un mode de réalisation, les moyens de rigidification 60 sont venus de matière avec la plaquette 20, et s'attachent au pont de liaison 50. Dans un autre mode de réalisation les moyens de rigidifications 60 sont des inserts placés dans la partie inférieure de la plaquette 20, et reliés au pont de liaison 50

Un autre objet de l'invention est un procédé de fabrication du conformateur narinaire 1. De préférence, le conformateur narinaire 1 est fabriqué par moulage par injection. Avantageusement, le matériau employé est du silicone.

Dans un mode de réalisation particulièrement avantageux, le moule utilisé pour le moulage par injection est lui-même fabriqué par fabrication additive. Ce mode de fabrication permet éventuellement la réalisation de moules sur mesure.

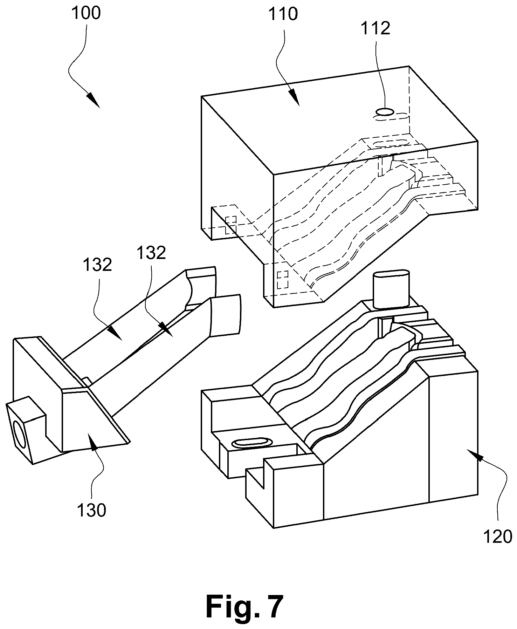

Un exemple de moule utilisé pour la fabrication du conformateur narinaire 1 est illustré à la figure 7. Le moule 100 comprend deux parties externes 110 et 120 et une partie interne 130. La partie 110 est la partie supérieure, et comprend un orifice 112 pour l'injection du silicone (ou autre matériau approprié). La partie 120 est la partie inférieure du moule. Le moule 100 comprend également une partie interne 130, comprenant en particulier deux pattes 132 autour desquelles vont être moulés les tubes 10 du conformateur narinaire 1.

**[Table 1].**

| | |
|---|---|
| 1 | Conformateur narinaire |
| 10 | Tube destiné à être introduit dans une narine |
| 12 | Protubérance arrière du tube 10 destinée à être reçue par un cornet du nez d'un sujet |
| 14 | Protubérance arrière du tube 10 destinée à être reçue par un cornet du nez d'un sujet |
| 16 | Protubérance avant du tube 10 destinée à être reçue par la partie avant supérieure du nez d'un sujet |
| 18 | Décrochement à l'extrémité inférieure du tube 10, avec angle adouci |
| 20 | Plaquette d'appui sur le philtrum |
| 30 | Languette latérale externe |
| 40 | Languette avant externe |
| 50 | Pont de liaison entre les tubes 10 |
| 60 | Moyen de rigidification |
| 100 | Moule pour la fabrication du conformateur narinaire 1 |
| 110 | Partie supérieure du moule |
| 112 | Orifice d'injection |
| 120 | Partie inférieure du moule |
| 130 | Partie du moule destinée à former les tubes 10 |
| 132 | Pattes |

L'invention n'est pas limitée aux modes de réalisation présentés et d'autres modes de réalisation apparaîtront clairement à l'homme du métier.

Notamment, on peut envisager que le conformateur narinaire 1 ne comporte pas de plaquette 20. Dans ce cas, le seul fait que les tubes soient pourvus d'une ou deux protubérances de calage 12, 14 permet de fournir un conformateur narinaire assurant une retenue meilleure dans le nez, en particulier qu ne requiert pas de suture du conformateur dans le nez.

En d'autres termes, le conformateur narinaire 1 est configuré pour être introduit dans le nez d'un sujet et comprend :
- deux tubes 10 aptes à être introduits chacun dans une narine du nez, reliés entre eux à leurs extrémités inférieures par un pont de liaison 50 et,
- dans lequel chaque tube 10 comprend dans sa partie arrière au moins une protubérance 12, 14 de calage du tube 10 dans les fosses nasales du sujet, de préférence deux protubérance 12, 14 de calage dans les fosses nasales du sujet.

Un conformateur narinaire de ce type est utile par exemple pour des rhinoplasties dans lesquelles aucun point de suture n'est posé sur le philtrum. Lorsque le conformateur est en place dans les narines, la protubérance est positionnée sous le cornet, ce qui permet un positionnement et un maintien optimal du conformateur. En effet, on a constaté sur les conformateurs existants, en particulier celui proposé dans la publication RU2477088, qui ne sont pas pourvus de ces protubérances, que le conformateur ne tient pas bien en place dans les narines car il a tendance à glisser vers le bas, ce qui oblige souvent à le suturer dans le nez.

Dans un autre mode de réalisation, le conformateur narinaire 1 ne comporte pas de plaquette 20, mais comporte des languettes externes de maintien du conformateur sur le nez du sujet. Dans un tel mode de réalisation, le conformateur narinaire 1 est configuré pour être introduit dans le nez d'un sujet et comprend :
- deux tubes 10 aptes à être introduits chacun dans une narine du nez, reliés entre eux à leurs extrémités inférieures par un pont de liaison 50 et
- des languettes externes 30,40 de maintien du conformateur sur le nez, s'étendant vers le haut depuis l'extrémité inférieure des tubes, de préférence deux languettes latérales 30 destinées à se plaquer sur les ailes du nez et/ou une languette avant 40 destinée à se plaquer sur la pointe du nez.

Un conformateur narinaire de ce type est utile par exemple pour des rhinoplasties dans lesquelles aucun point de suture n'est posé sur le philtrum. La présence des languettes 30, 40 améliore la tenue du conformateur, et permet également d'appliquer une pression sur les différentes couches tissulaires des ailes nasales, dans le cas où elles sont décollées durant la chirurgie, améliorant ainsi la cicatrisation et permettant de maintenir la forme donnée par le chirurgien pendant l'intervention. Ces languettes 30, 40 peuvent également servir à porter des points de sutures posés à travers les alaires, améliorant encore plus le contact des différentes couches tissulaires des ailes nasales.

Il est en outre possible de combiner les deux modes de réalisation ci-dessus, un objet de l'invention étant alors un conformateur narinaire 1 comprenant :
- deux tubes 10 aptes à être introduits chacun dans une narine du nez, reliés entre eux à leurs extrémités inférieures par un pont de liaison 50 et,
- dans lequel chaque tube 10 comprend dans sa partie arrière au moins une protubérance 12, 14 de calage du tube 10 dans les fosses nasales du sujet, de préférence deux protubérance 12, 14 de calage dans les fosses nasales du sujet.
- des languettes externes 30,40 de maintien du conformateur sur le nez, s'étendant vers le haut depuis l'extrémité inférieure des tubes, de préférence deux languettes latérales 30 destinées à se plaquer sur les ailes du nez et/ou une languette avant 40 destinée à se plaquer sur la pointe du nez.

On peut envisager d'autres combinaisons ou suppressions de caractéristiques des différents modes de réalisation.

## Revendications

1. Conformateur narinaire (1) configuré pour être introduit dans le nez d'un sujet **caractérisé en ce qu'**il comprend :
- deux tubes (10) aptes à être introduits chacun dans une narine du nez, reliés entre eux à leurs extrémités inférieures par un pont de liaison (50) et
- une plaquette (20) attachée au pont de liaison (50) et s'étendant à l'opposé du pont de liaison (50) par rapport aux tubes (10), la plaquette (20) étant configurée pour exercer une pression sur le philtrum du sujet lorsque les tubes (10) sont introduits chacun dans une narine du nez.

2. Conformateur narinaire (1) selon la revendication précédente, dans lequel la plaquette (20) est attachée au pont de liaison (50) en étant déformable entre :
- une position de repos, dans laquelle la plaquette (20) s'étend vers l'arrière du conformateur narinaire (1), en regard des tubes (10) avant la mise en place du conformateur, et
- une position d'utilisation, lorsque le conformateur narinaire (1) est introduit dans le nez, dans laquelle la plaquette s'étend vers l'avant du conformateur narinaire (1).

3. Conformateur narinaire (1) selon la revendication précédente, comprenant des moyens de rigidification (60), exerçant un rappel de la plaquette (20) vers sa position de repos, par exemple un insert.

4. Conformateur narinaire (1) selon l'une quelconque des revendications précédentes dans lequel chaque tube (10) comprend dans sa partie avant supérieure au moins une protubérance (16) de positionnement du tube (10) dans la narine.

5. Conformateur narinaire (1) selon l'une quelconque des revendications précédentes dans lequel chaque tube (10) comprend dans sa partie arrière au moins une protubérance (12,14) de calage du tube (10) dans les fosses nasales du sujet, de préférence deux protubérances (12,14) de calage dans les fosses nasales du sujet.

6. Conformateur narinaire (1) selon l'une quelconque des revendications précédentes dans lequel l'extrémité inférieure de chaque tube (10) présente un décrochement de façon à laisser un espace entre l'extrémité inférieure du tube (10) et le bas de la narine, de préférence, ce décrochement présente la forme générale d'une marche d'escalier, pourvue de congés permettant d'adoucir les angles, reliant l'arrière et la partie inférieure du tube (10).

7. Conformateur narinaire (1) selon l'une quelconque des revendications précédentes comprenant en outre des languettes externes (30,40) de maintien du conformateur sur le nez, s'étendant vers le haut depuis l'extrémité inférieure des tubes, de préférence deux languettes latérales (30) destinées à se plaquer sur les ailes du nez et/ou une languette avant (40) destinée à se plaquer sur la pointe du nez.

8. Conformateur narinaire (1) selon l'une quelconque des revendications précédentes, comprenant du silicone, de préférence constitué de silicone.

9. Conformateur narinaire (1) selon l'une des revendications précédentes, dans lequel la plaquette (20) est fabriquée en un matériau plus rigide que le matériau des tubes (10).

10. Conformateur narinaire (1) selon l'une des revendications 3 à 9, dans lequel les éléments de rigidification (60) sont fabriqués en un matériau plus rigide que le matériau des tubes (10).

11. Conformateur narinaire (1) selon l'une ou quelconque des revendications précédentes, comprenant des couleurs de matériau différentes.

12. Conformateur narinaire (1) selon l'une quelconque des revendications précédentes, disposant d'une longueur de pont de liaison variable.

13. Procédé de fabrication d'un conformateur narinaire (1) selon l'une des revendications précédentes, dans lequel le conformateur narinaire (1) est fabriqué par moulage par injection, le moule étant de préférence réalisé par fabrication additive.

## Patentansprüche

1. Nasenformer (1), der so konfiguriert ist, dass er in die Nase eines Subjekts eingeführt werden kann, **dadurch gekennzeichnet, dass** er umfasst:
- zwei Röhren (10), die geeignet sind, jeweils in ein Nasenloch der Nase eingeführt zu werden, und die an ihren unteren Enden durch eine Verbindungsbrücke (50) miteinander verbunden sind, und
- ein Plättchen (20), das an der Verbindungsbrücke (50) befestigt ist und sich in Bezug auf die Röhren (10) gegenüber der Verbindungsbrücke (50) erstreckt, wobei das Plättchen (20) so konfiguriert ist, dass es einen Druck auf das Philtrum des Subjekts ausübt, wenn die Röhren (10) jeweils in ein Nasenloch der Nase eingeführt werden.

2. Nasenformer (1) nach dem vorhergehenden Anspruch, bei dem das Plättchen (20) an der Verbindungsbrücke (50) befestigt ist, indem es verformbar ist zwischen:
- einer Ruheposition, in der sich das Plättchen (20) vor dem Einsetzen des Nasenformers (1) gegenüber den Röhren (10) nach hinten vom Nasenformer (1) erstreckt, und
- einer Gebrauchsposition, wenn der Nasenformer (1) in die Nase eingeführt wird, in der sich das Plättchen nach vorne vom Nasenbeinformer (1) erstreckt.

3. Nasenformer (1) nach dem vorhergehenden Anspruch, mit Versteifungsmitteln (60), die eine Rückstellung des Plättchens (20) in seine Ruhestellung bewirken, z. B. ein Einsatz.

4. Nasenformer (1) nach einem der vorhergehenden Ansprüche, bei dem jede Röhre (10) in seinem vorderen oberen Teil mindestens einen Vorsprung (16) zur Positionierung der Röhre (10) im Nasenloch aufweist.

5. Nasenformer (1) nach einem der vorhergehenden Ansprüche, bei dem jede Röhre (10) in seinem hinteren Teil mindestens einen Vorsprung (12, 14) zum Verkeilen der Röhre (10) in den Nasenhöhlen der Person, vorzugsweise zwei Vorsprünge (12, 14) zum Verkeilen in den Nasenhöhlen der Person, aufweist.

6. Nasenformer (1) nach einem der vorhergehenden Ansprüche, bei dem das untere Ende jeder Röhre (10) eine Aussparung aufweist, so dass ein Raum zwischen dem unteren Ende der Röhre (10) und dem unteren Teil des Nasenlochs verbleibt, vorzugsweise weist diese Aussparung die allgemeine Form einer Treppenstufe auf, die mit Verrundungen versehen ist, die es ermöglichen, die Winkel zu mildern, und die den hinteren und unteren Teil der Röhre (10) miteinander verbindet.

7. Nasenformer (1) nach einem der vorhergehenden Ansprüche, ferner umfassend äußere Laschen (30, 40) zum Halten des Formers auf der Nase, die sich vom unteren Ende der Röhren nach oben erstrecken, vorzugsweise zwei seitliche Laschen (30), die dazu bestimmt sind, sich an die Nasenflügel anzulegen, und/oder eine vordere Lasche (40), die dazu bestimmt ist, sich an die Nasenspitze anzulegen.

8. Nasenformer (1) nach einem der vorhergehenden Ansprüche, enthaltend Silikon, vorzugsweise bestehend aus Silikon.

9. Nasenformer (1) nach einem der vorhergehenden Ansprüche, wobei das Plättchen (20) aus einem Material hergestellt ist, das steifer ist als das Material der Röhren (10).

10. Nasenformer (1) nach einem der Ansprüche 3 bis 9, wobei die Versteifungsmittel (60) aus einem Material hergestellt sind, das steifer ist als das Material der Röhren (10).

11. Nasenformer (1) nach einem oder mehreren der vorhergehenden Ansprüche, umfassend unterschiedliche Materialfarben.

12. Nasenformer (1) nach einem der vorhergehenden Ansprüche, der über eine variable Länge der Verbindungsbrücke verfügt.

13. Verfahren zur Herstellung eines Nasenformers (1) nach einem der vorhergehenden Ansprüche, wobei der Nasenformer (1) durch Spritzgießen hergestellt wird, wobei die Form vorzugsweise durch additive Fertigung hergestellt wird.

## Claims

1. Nasal shaper (1) configured to be introduced into the nose of a subject, **characterised in that** it comprises:
- two tubes (10) suitable for each being introduced into a nostril of the nose, connected together at their lower ends by a connecting bridge (50) and
- a plate (20) attached to the connecting bridge (50) and extending opposite the connecting bridge (50) relative to the tubes (10), the plate (20) being configured to exert a pressure on the philtrum of the subject when the tubes (10) are each introduced into a nostril of the nose.

2. Nasal shaper (1) according to the preceding claim, wherein the plate (20) is attached to the connecting bridge (50), being deformable between:
- a rest position, in which the plate (20) extends towards the rear of the nasal shaper (1), opposite the tubes (10) before positioning the shaper, and
- a use position, when the nasal shaper (1) is introduced into the nose, in which the plate extends towards the front of the nasal shaper (1).

3. Nasal shaper (1) according to the preceding claim, comprising stiffening means (60), exerting a force to return the plate (20) to its rest position, for example an insert.

4. Nasal shaper (1) according to any one of the preceding claims, wherein each tube (10) comprises in its upper front part at least one protuberance (16) to position the tube (10) in the nostril.

5. Nasal shaper (1) according to any one of the preceding claims, wherein each tube (10) comprises in its rear part at least one blocking protuberance (12, 14) to block the tube (10) in the nasal cavities of the subject, preferably two blocking protuberances (12, 14) in the nasal cavities of the subject.

6. Nasal shaper (1) according to any one of the preceding claims, wherein the lower end of each tube (10) has a recess so as to leave a space between the lower end of the tube (10) and the bottom of the nostril, preferably, this recess has the general shape of a step, provided with fillets to soften the corners, connecting the rear and the lower part of the tube (10).

7. Nasal shaper (1) according to any one of the preceding claims, further comprising external tabs (30, 40) to hold the shaper on the nose, extending upwards from the lower ends of the tubes, preferably two lateral tabs (30) intended to press against the wings of the nose and/or one front tab (40) intended to press against the tip of the nose.

8. Nasal shaper (1) according to any one of the preceding claims, comprising silicone, preferably made of silicone.

9. Nasal shaper (1) according to one of the preceding claims, wherein the plate (20) is made of a material which is stiffer than the material of the tubes (10).

10. Nasal shaper (1) according to one of claims 3 to 9, wherein the stiffening elements (60) are made of a material which is stiffer than the material of the tubes (10).

11. Nasal shaper (1) according to any one of the preceding claims, comprising materials of different colours.

12. Nasal shaper (1) according to any one of the preceding claims, having a connecting bridge of variable length.

13. Method for manufacturing a nasal shaper (1) according to one of the preceding claims, wherein the nasal shaper (1) is manufactured by injection moulding, the mould preferably being manufactured by additive manufacturing.
